# EUROPÄISCHE PATENTSCHRIFT

(11) **EP 0 944 657 B1**
(45) Veröffentlichungstag und Bekanntmachung des Hinweises auf die Patenterteilung: **06.02.2002**
(21) Anmeldenummer: 97952006.1
(22) Anmeldetag: 28.11.1997
(51) Int. Cl.: C08F 271/00, C08F 8/12, B01F 17/00, C09B 67/00, C11D 3/37, D21H 17/34, C09K 17/20, C05G 3/00

(54) **AMPHIPHILE PFROPFPOLYMERISATE AUF BASIS VON N-VINYLCARBONSÄUREAMID-EINHEITEN ENTHALTENDEN PFROPFGRUNDLAGEN, VERFAHREN ZU IHRER HERSTELLUNG UND IHRE VERWENDUNG**
AMPHIPHILIC GRAFT POLYMERS BASED ON GRAFT BASES CONTAINING N-VINYLCARBOXYLIC ACID UNITS, PROCESS FOR THEIR PREPARATION AND THEIR USE
POLYMERISATS GREFFES AMPHIPHILES AVEC DES BASES GREFFEES CONTENANT DES UNITES N-AMIDE D'ACIDE CARBOXYLIQUE VINYLIQUE, PROCEDES PERMETTANT DE LES PREPARER ET LEUR UTILISATION

(30) Priorität: 10.12.1996 DE 19651243
(43) Veröffentlichungstag der Anmeldung: 29.09.1999
(73) Patentinhaber: BASF AKTIENGESELLSCHAFT, 67056 Ludwigshafen (DE)
(72) Erfinder: NEGELE, Anton, D-67146 Deidesheim (DE); RÜBENACKER, Martin, D-67122 Altrip (DE); UTECHT, Jens, D-68809 Neulussheim (DE); MEIXNER, Hubert, D-67069 Ludwigshafen (DE)
(86) Internationale Anmeldenummer: EP9706652
(87) Internationale Veröffentlichungsnummer: WO9825981

(56) Entgegenhaltungen:
- WO-A-94/08092
- WO-A-95/25759
- GB-A- 1 551 513
- US-A- 3 249 571

## Beschreibung

Die Erfindung betrifft amphiphile Pfropfpolymerisate auf Basis von N-Vinylcarbonsäureamid-Einheiten enthaltenden Pfropfgrundlagen, Verfahren zu ihrer Herstellung und ihre Verwendung als Verfestigungsmittel für Papier, als Fixiermittel für wasserlösliche und wasserunlösliche Störstoffe bei der Herstellung von Papier, als Zusatz zu Wasch- und Reinigungsmitteln, als Dispergiermittel für anorganische und organische Pigmente, Farbstoffe, Beton- und Pflanzenschutzmittel, als Beschichtungsmaterial für Düngemittel und Pflanzenschutzmittel, als Bodenpflegemittel, als Schutzkolloid für wäßrige Polymerdispersionen, als Verdicker für Kosmetikformulierungen, als Conditioner für Hautpflegemittel und als Bestandteil von haarkosmetischen Zubereitungen und von kosmetischen Zubereitungen für die Mundpflege.

Aus der DE-A-27 11 458 ist ein Verfahren zur Verlängerung der Haltbarkeit eines Fäulnis verhindernden Schutzanstrichfilms bekannt, wobei man auf den Schutzanstrichfilm zur Bildung eines Schutzüberzugs eine Zusammensetzung aufbringt, die ein Polymer enthält, das durch Polymerisation von mindestens einem Vinylmonomeren aus der Gruppe Methacrylsäureester, Acrylsäureester, Acrylnitril, Styrol, α-Methylstyrol und Vinylacetat in Anwesenheit eines hydrophilen Polymeren aus der Gruppe Polyvinylalkohol, Polyethylenglykol, Polypropylenglykol, Polyvinylpyrrolidon oder Polyvinylamin hergestellt wurde. Die Pfropfpolymeren werden durch radikalisch initiierte Polymerisation der Monomeren in Gegenwart der genannten Polymeren in einem organischen Lösemittel wie Toluol hergestellt.

Aus der US-A-4 238 579 sind Copolymerisate bekannt, die Vinylamin- und Styroleinheiten enthalten. Sie werden durch radikalisch initiierte Copolymerisation von Vinylacetamid und Styrol in Substanz oder in Lösung und anschließende teilweise oder vollständige Hydrolyse der Amidgruppen des einpolymerisierten N-Vinylacetamids unter Bildung von Aminogruppen hergestellt. Die Polymeren werden beispielsweise als Beschichtungsmittel oder als Härter für Epoxidharze verwendet.

Aus der WO-A-95/25759 sind Pfropfpolymerisate bekannt, bei denen die Pfropfgrundlage ein Polymerisat ist, das jeweils mindestens 5 Gew.-% Einheiten der Formeln 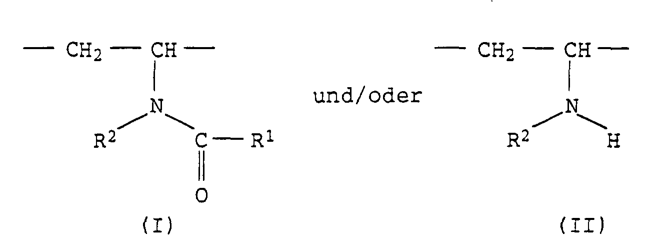 enthält, wobei R¹, R² = H oder C₁- bis C₆-Alkyl bedeuten. Auf die Pfropfgrundlage sind monoethylenisch ungesättigte Monomere im Gewichtsverhältnis 100 : 1 bis 1 : 100 aufgepfropft.

Auf die Pfropfgrundlage werden bevorzugt Monomere aus der Gruppe N-Vinylpyrrolidon, N-Vinylcaprolactam, N-Vinylimidazol, Acrylsäure, Methacrylsäure, Acrylamid, Acrylnitril und Vinylacetat aufgepfropft. Die Pfropfpolymerisate werden beispielsweise als Dispergiermittel für Pigmente, als Zusatz zu Wasch- und Reinigungsmitteln, als Verfestiger für Papier und als Mittel für die Bodenverbesserung und Düngemittelkompaktierung verwendet.

Soweit die oben beschriebenen Pfropfpolymerisate bei der Herstellung von Papier eingesetzt werden, läßt ihre Wirksamkeit, insbesondere die Fixierwirkung für lösliche und unlösliche Störstoffe bei der Papierherstellung noch Wünsche offen.

Der vorliegenden Erfindung liegt die Aufgabe zugrunde, neue Pfropfpolymerisate zur Verfügung zu stellen, die insbesondere bei der Herstellung von Papier lösliche und unlösliche Störstoffe wie Pitch wirksam auf dem gebildetem Papierblatt fixieren.

Die Aufgabe wird erfindungsgemäß gelöst mit amphiphilen Pfropfpolymerisaten auf Basis von N-Vinylcarbonsäureamid-Einheiten enthaltenden Pfropfgrundlagen, wobei die amphiphilen Pfropfpolymerisate erhältlich sind durch radikalische Polymerisation von Monomermischungen aus
(a) Styrol, C₁ bis C₂-Alkylstyrol und/oder Vinyltoluol und gegebenenfalls
(b) anderen damit copolymerisierbaren monoethylenisch ungesättigten Monomeren auf ein Polymerisat, das mindestens 5 Gew.-% Einheiten der Formel 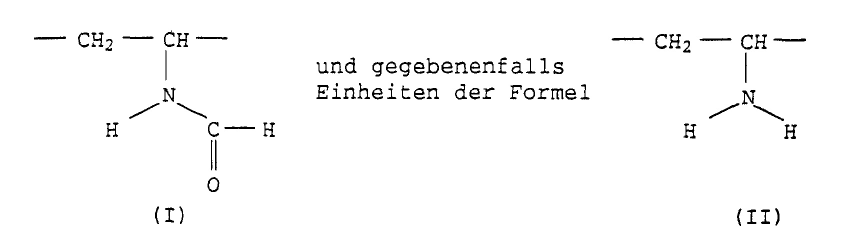 enthält, als Pfropfgrundlage, im Gewichtsverhältnis 1:99 bis 99:1 und anschließende 5 bis 100%ige Abspaltung der Formylgruppen unter Bildung von Aminogruppen aus den Pfropfpolymerisaten.

Gegenstand der Erfindung ist außer dem ein Verfahren zur Herstellung der obengenannten amphiphilen Pfropfpolymerisate, wobei man Monomermischungen aus
(a) Styrol, C₁- bis C₂-Alkylstyrol und/oder Vinyltoluol und gegebenenfalls
(b) anderen damit copolymerisierbaren monoethylenisch ungesättigten Monomeren
radikalisch in Gegenwart von Polymerisaten, die mindestens 5 Gew. -% N-Vinylformamid-Einheiten enthalten, als Pfropfgrundlage im Gewichtsverhältnis 1:99 bis 99:1 polymerisiert und im Anschluß an die Pfropfpolymerisation 5 bis 100% der Formylgruppen aus den N-Vinylformamid-Einheiten unter Bildung von Aminogruppen abspaltet.

Die oben beschriebenen amphiphilen Pfropfpolymeren werden mit besonderem Vorteil als Fixiermittel für lösliche und kolloidale Störstoffe bei der Papierherstellung verwendet. Solche Störstoffe sind beispielsweise in Form von Huminsäuren, Ligninsulfonat, Kieselsäuren oder Holzextrakt im Papierstoff enthalten. Die amphiphilen Pfropfpolymeren werden zur Fixierung von unlöslichen, lipophil/hydrophoben Störstoffen, sogenannten Stickies oder White Pitch verwendet. Außerdem eignen sie sich als Dispergiermittel in der Papierstreicherei. Von den oben beschriebenen Anwendungen ist außerdem ihr Einsatz als Schutzkolloid für Acrylat-, Styrol- und Butadiendispersionen und der Zusatz zu Wasch- und Reinigungsmitteln von Interesse.

Als Pfropfgrundlage dienen Polymerisate, die mindestens 5 Gew.-% Einheiten der oben angegebenen Formel I einpolymerisiert enthalten. Als Pfropfgrundlage kann man auch hydrolisierte Poly-N-vinyl formamide einsetzen, die durch Behandlung von Poly-N-vinyl formamiden mit Säuren oder Basen zugänglich sind, und die neben den Einheiten der Formel I Einheiten der Formel 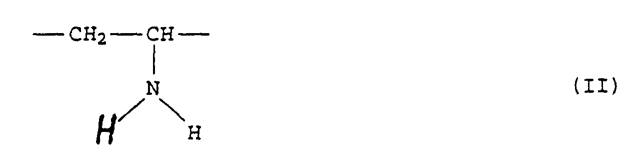 enthalten. Die Pfropfgrundlage kann gegebenenfalls bis zu 95 Gew.-% an Einheiten der Formel II enthalten.

Den Einheiten der Formel I liegt N-Vinylformamid als Monomer zugrunde. Diese Monomeren können zur Herstellung von Polymerisaten allein, in Mischung miteinander, z.B. Mischungen aus N-Vinylformamid und N-Vinylacetamid oder zusammen mit anderen copolymerisierbaren Monomeren eingesetzt werden. Verfahren zur Herstellung solcher Homo- und Copolymerisate mit anderen Monomeren sind bekannt, vgl. EP-B-0 071 050, EP-B-0 215 387, EP-B-0 251 182, EP-A-0 528 409 und EP-A-0 337 310.

Als andere, mit N-Vinyl formamid copolymerisierbare Monomere eignen sich beispielsweise monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure. Aus dieser Gruppe von Monomeren verwendet man vorzugsweise Acrylsäure, Methacrylsäure, Maleinsäure oder Mischungen der genannten Carbonsäuren. Die monoethylenisch ungesättigten Carbonsäuren können in Form der freien Säure und - soweit vorhanden - der Anhydride oder in partiell oder in vollständig neutralisierter Form bei der Copolymerisation eingesetzt werden. Um diese Monomeren zu neutralisieren, verwendet man vorzugsweise Alkalimetall- oder Erdalkalimetallbasen, Ammoniak oder Amine, z.B. Natronlauge, Kalilauge, Soda, Pottasche, Natriumhydrogencarbonat, Magnesiumoxid, Calciumhydroxid, Calciumoxid, gasförmiges oder wäßriges 5 Ammoniak, Triethylamin, Ethanolamin, Diethanolamin, Triethanolamin, Morpholin, Diethylentriamin oder Tetraethylenpentamin.

Weitere geeignete Comonomere zur Herstellung der Pfropfgrundlage sind beispielsweise die Ester, Amide und Nitrile der oben angegebenen Carbonsäuren, z.B. Acrylsäuremethylester, Acrylsäureethylester, Methacrylsäuremethylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Acrylamid, Methacrylamid, N,N-Dimethylacrylamid, N-tert.-butylacrylamid, Acrylnitril, Methacrylnitril, Dimethylaminoethylacrylat, Diethylaminoethylacrylat, Diethylaminoethylmethacrylat, sowie die Salze der zuletzt genannten Monomeren mit Carbonsäuren oder Mineralsäuren sowie die quaternierten Produkte.

Außerdem eignen sich als andere copolymerisierbare Monomere Acrylamidoglykolsäure, Vinylsulfonsäure, Allylsulfonsäure, Methallylsulfonsäure, Styrolsulfonsäure, Acrylsäure-(3-sulfopropyl)ester, Methacrylsäure(3-sulfopropyl)ester und Acrylamidomethylpropansulfonsäure sowie Phosphonsäuregruppen enthaltende Monomere, wie Vinylphosphonsäure, Allylphosphonsäure und Acrylamidomethanpropanphosphonsäure. Auch diese Säuregruppen enthaltenden Monomeren können in Form der freien Säuren oder in partiell oder vollständig neutralisierter Form eingesetzt werden. Geeignete Basen für die Neutralisation wurden bereits oben genannt. Vorzugsweise setzt man Natronlauge oder Ammoniak ein.

Weitere geeignete, mit N-Vinylformamid copolymerisierbare Verbindungen sind N-Vinylpyrrolidon, N-Vinylcaprolactam, N-Vinylimidazol, N-Vinyl-2-methylimidazol, N-Vinyl-4-methylimidazol, Diallylammoniumchlorid, Vinylacetat und Vinylpropionat. Es ist selbstverständlich auch möglich, Mischungen der genannten Monomeren einzusetzen.

Die als Pfropfgrundlage eingesetzten Copolymeren enthalten mindestens 5, meistens mindestens 20 und bevorzugt mindestens 50 Gew.-% N-Vinylamide einpolymerisiert.

Die Herstellung der als Pfropfgrundlage eingesetzten Copolymerisate erfolgt nach bekannten Verfahren, z.B. der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Die Homo- und Copolymeren von N-Vinylcarbonsäureamiden besitzen K-Werte von mindestens 7 bis 300, vorzugsweise 10 bis 250. Die K-Werte werden bestimmt nach H. Fikentscher, Cellulose-Chemie, Band 13, 58 bis 64 und 71 bis 74 (1932) in wäßriger Lösung bei 25°C und bei Konzentrationen, die je nach K-Wert-Bereich zwischen 0,1 % und 5 % liegen. Aus den oben beschriebenen Homo- und Copolymerisaten erhält man durch teilweise Abspaltung der Gruppe 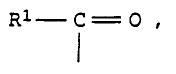 in der R¹ = H ist, unter Bildung von Aminbzw. Ammoniumgruppen als Pfropfgrundlage zu verwendende gegebenenfalls hydrolysierte Copolymerisate, die Einheiten der Formeln I und II aufweisen. Wenn hydrolysierte Copolymerisate der N-Vinylcarbonsäureamide als Pfropfgrundlage eingesetzt werden, können auch die eingesetzten Comonomeren je nach gewählter Hydrolysebedingung chemisch verändert werden, z.B. entstehen aus Vinylacetat-Einheiten Vinylalkohol-Einheiten und aus Acrylsäuremethylester-Einheiten Acrylsäure-Einheiten und aus Acrylnitril-Einheiten Acrylamid- bzw. Acrylsäure-Einheiten.

Als Hydrolysemittel eignen sich Mineralsäuren, wie Halogenwasserstoffe, die gasförmig oder in wäßriger Lösung eingesetzt werden können. Vorzugsweise verwendet man Salzsäure, Schwefelsäure, Salpetersäure und Phosphonsäure sowie organische Säuren, wie C₁- bis C₅-Carbonsäuren und aliphatische oder aromatische Sulfonsäuren. Pro Formylgruppenäquivalent, das aus den einpolymerisierten Einheiten I abgespalten werden soll, kann man z.B. 0,05 bis 2, vorzugsweise 1 bis 1,5 Moläquivalente einer Säure einsetzen.

Die Hydrolyse der einpolymerisierten Einheiten der Struktur I kann auch mit Hilfe von Basen vorgenommen werden, z.B. von Metallhydroxiden, insbesondere von Alkalimetall- und Erdalkalimetallhydroxiden. Vorzugsweise verwendet man Natriumhydroxid oder Kaliumhydroxid. Die Hydrolyse kann gegebenenfalls auch in Gegenwart von Ammoniak oder Aminen durchgeführt werden. Die Vinylamineinheiten können in Form der freien Amine oder auch als Ammoniumsalze zur Pfropfung eingesetzt werden.

Die oben beschriebenen Polymerisate, die Einheiten der Formel I und gegebenenfalls II enthalten, werden der Pfropfung mit Styrol, Alkylstyrolen und/oder Vinyltoluol unterzogen. Nach der Pfropfungsreaktion wird das resultierende Pfropfpolymere unter den oben genannten Bedingungen nachträglich hydrolysiert werden. Danach liegen je nach den Hydrolysebedingungen funktionelle Gruppen der Formeln I und/oder II vor.

Zur Pfropfung wird vorzugsweise Styrol, Alkylstyrol, wie α-Methylstyrol und α-Ethylstyrol, und/oder Vinyltoluol als nicht hydrolysierbares und hydrophobes Monomer verwendet. Es ist selbstverständlich auch möglich, Mischungen von Styrol und/oder α-Alkylstyrolen und oder Vinyltoluol mit allen in der WO-A-95/25759 bereits genannten und zur Pfropfung geeigneten Monomeren einzusetzen. Diese Monomeren sind oben als Comonomere für die Herstellung der Pfropfgrundlage beispielhaft genannt. Darunter fallen in bevorzugten Maße Acrylnitril, Acrylsäure-n-butylester, Methacrylsäure-n-butylester, Dimethylaminoethylacrylat oder 2-Ethylhexylacrylat.

Bevorzugt werden solche amphiphilen Pfropfpolymerisate, die dadurch erhältlich sind, daß man auf eine Pfropfgrundlage aus einem Homopolymerisat aus N-Vinylformamid oder aus einem Copolymerisat aus N-Vinylformamid und N-Vinylcaprolactam Monomermischungen aus
(a) 5 bis 100 Gew.-% Styrol, C₁- bis C₂-Alkylstyrol und/oder Vinyltoluol und
(b) 0 bis 95 Gew.-% anderen copolymerisierbaren monoethylenisch ungesättigten Monomeren aufpfropft.

Besonders bevorzugt sind amphiphile Pfropfpolymerisate zu deren Herstellung man auf eine Pfropfgrundlage aus Poly-N-Vinylformamid Styrol aufpfropft und im Anschluß an die Pfropfpolymerisation 5 bis 100 % der Formylgruppen unter Bildung von Aminogruppen aus dem Pfropfpolymerisat abspaltet.

Zur Herstellung der Pfropfpolymerisate wird Styrol, Alkylstyrol und/oder Vinyltoluol in Gegenwart der Pfropfgrundlage vorzugsweise in wäßriger Lösung radikalisch polymerisiert. Eine bevorzugte Art der Herstellung der Pfropfcopolymerisate ist die Lösungspolymerisation, wobei die als Pfropfgrundlage eingesetzten Polymerisate, vorzugsweise in gelöster Form vorliegen, Das aufzupfropfende Styrol und/oder Alkylstyrol und gegebenenfalls weitere zugesetzte Monomere werden dann der Polymerlösung entweder langsam oder auf einmal zugesetzt und in der Polymerlösung suspendiert. Für die Lösungspolymerisation eignen sich beispielsweise inerte Lösemittel, wie Methanol, Ethanol, Isopropanol, n-Propanol, n-Butanol, sek.-Butanol, Tetrahydrofuran, Dioxan, sowie Mischungen der genannten inerten Lösemittel. Bevorzugt ist die Lösungspolymerisation in Wasser oder in Mischungen aus Wasser und Alkoholen. Die Pfropfcopolymerisation kann kontinuierlich oder diskontinuierlich durchgeführt werden.

Die Pfropfcopolymerisate werden im allgemeinen unter Mitverwendung von radikalischen Initiatoren hergestellt. Als radikalbildende Initiatoren sind vorzugsweise alle diejenigen Verbindungen geeignet, die bei der jeweils gewählten Polymerisationstemperatur eine Halbwertszeit von weniger als 3 Stunden aufweisen. Falls man die Polymerisation zunächst bei niedriger Temperatur startet und bei höherer Temperatur zu Ende fährt, so ist es zweckmäßig, mit mindestens zwei bei verschiedenen Temperaturen zerfallenden Initiatoren zu arbeiten, nämlich zunächst einen bereits bei niedrigerer Temperatur zerfallenden Initiator für den Start der Polymerisation einzusetzen und dann die Hauptpolymerisation mit einem Initiator zu Ende zu führen, der bei höherer Temperatur zerfällt. Man kann wasserlösliche sowie wasserunlösliche Initiatoren oder Mischungen von wasserlöslichen und wasserunlöslichen Initiatoren einsetzten. Die in Wasser unlöslichen Initiatoren sind dann in der organischen Phase löslich. Für die im folgenden angegebenen Temperaturbereiche kann man beispielsweise die dafür aufgeführten Initiatoren verwenden.

### Temperatur: 30 bis 60°C:

Acetylcyclohexansulfonylperoxid, Diacetylperoxydicarbonat, Dicyclohexylperoxydicarbonat, Di-2-ethylhexylperoxydicarbonat, tert.-Butylperneodecanoat, 2,2'-Azobis-(4-methoxy-2,4-dimethylvaleronitril), 2,2'-Azobis(2-methyl-N-phenylpropionamidin)dihydrochlorid, 2,2'-Azobis-(2-methylpropionamidin)dihydrochlorid.

### Temperatur: 60 bis 80°C:

tert.-Butylperpivalat, Dioctanoxyperoxid, Dilauroylperoxid, 2,2'-Azobis-(2,4-dimethylvaleronitril).

### Temperatur: 80 bis 100°C:

Dibenzoylperoxid, tert.-Butylper-2-ethylhexanoat, tert.-Butylpermaleinat, 2,2'-Azobis-(isobutyronitril), Dimethyl-2,2'-azobisisobutyrat, Natriumpersulfat, Kaliumpersulfat, Ammoniumpersulfat.

### Temperatur: 100 bis 120°C:

Bis-(tert.-butylperoxy)-cyclohexan, tert.-Butylperoxyisopropylcarbonat, tert.-Butylperacetat, Wasserstoffperoxid.

### Temperatur: 120 bis 140°C:

2,2-Bis-(tert.-butylperoxy)-butan, Dicumylperoxid, Di-tert.-amylperoxid, Di-tert.-butylperoxid.

### Temperatur: > 140°C:

p-Menthanhydroperoxid, Pinanhydroperoxid, Cumolhydroperoxid und tert.-Butylhydroperoxid. Verwendet man zusätzlich zu den genannten Initiatoren noch Salze oder Komplexe von Schwermetallen, z.B. Kupfer-, Kobalt-, Mangan-, Eisen-, Vanadium-, Nickel- und Chromsalze oder organische Verbindungen, wie Benzoin, Dimethylanilin oder Ascorbinsäure, so können die Halbwertszeiten der angegebenen radikalbildenden Initiatoren verringert werden. So kann man beispielsweise tert.-Butylhydroperoxid unter Zusatz von 5 ppm Kupfer-II-acetylacetonat bereits so aktivieren, daß bereits bei 100°C polymerisiert werden kann. Die reduzierende Komponente von Redoxkatalysatoren kann auch beispielsweise von Verbindungen wie Natriumsulfit, Natriumbisulfit, Natriumformaldehydsulfoxylat und Hydrazin gebildet werden. Bezogen auf die bei der Polymerisation eingesetzten Monomeren verwendet man z.B. 0,01 bis 20, vorzugsweise 0,05 bis 10 Gew.-% eines Polymerisationsinitiators oder einer Mischung mehrerer Polymerisationsinitiatoren. Als Redoxkomponenten setzt man 0,01 bis 15 % der reduzierend wirkenden Verbindungen zu. Schwermetalle werden beispielsweise im Bereich von 0,1 bis 100 ppm, vorzugsweise 0,5 bis 10 ppm eingesetzt. Oft ist es von Vorteil, eine Kombination aus Peroxid, Reduktionsmittel und Schwermetall als Redoxkatalysator einzusetzen.

Die Copolymerisation von Styrol, Alkylstyrol und/oder Vinyltoluol und eventuellen weiteren Monomeren in Gegenwart der Pfropfgrundlage mit Einheiten der Formeln I und gegebenenfalls II kann auch durch Einwirkung von ultravioletter Strahlung, gegebenenfalls in Gegenwart von UV-Initiatoren, durchgeführt werden. Für das Polymerisieren unter Einwirkung von UV-Strahlen setzt man die dafür üblicherweise in Betracht kommenden Photoinitiatoren bzw. Sensibilisatoren ein. Hierbei handelt es sich beispielsweise um Verbindungen wie Benzoin und Benzoinether, α-Methylbenzoin oder α-Phenylbenzoin. Auch sogenannte Triplett-Sensibilisatoren, wie Benzyldiketale, können verwendet werden. Als UV-Strahlungsquellen dienen beispielsweise neben energiereichen UV-Lampen, wie Kohlenbogenlampen, Quecksilberdampflampen oder Xenonlampen auch UV-arme Lichtquellen, wie Leuchtstoffröhren mit hohem Blauanteil.

Um Pfropfpolymerisate mit niedrigem K-Wert herzustellen, wird die Pfropfcopolymerisation zweckmäßigerweise in Gegenwart von Reglern durchgeführt. Geeignete Regler sind beispielsweise Mercaptoverbindungen, wie Mercaptoethanol, Mercaptopropanol, Mercaptobutanol, Mercaptoessigsäure, Mercaptopropionsäure, Butylmercaptan und Dodecylmercaptan. Als Regler eignen sich außerdem Allylverbindungen, wie Allylalkohol, Aldehyde, wie Formaldehyd, Acetaldehyd, Propionaldehyd, n-Butyraldehyd und Isobutyraldehyd, Ameisensäure, Ammoniumformiat, Propionsäure, Hydrazinsulfat und Butenole. Falls die Polymerisation in Gegenwart von Reglern durchgeführt wird, benötigt man davon 0,05 bis 20 Gew.-%, bezogen auf die bei der Polymerisation eingesetzten Monomeren.

Üblicherweise wird die Pfropfpolymerisation bei Temperaturen von 20 bis 200°C durchgeführt, wobei man gegebenenfalls unter erhöhtem Druck arbeitet. Der bevorzugte Temperaturbereich liegt jedoch bei 30 bis 120°C.

Wenn die Reaktionen in Lösung durchgeführt werden, wählt man zweckmäßigerweise Konzentrationen zwischen 5 und 80 Gew.-%. Der bevorzugte Konzentrationsbereich liegt bei 10 bis 60 Gew.-%.

Eine bevorzugte Herstellungsart für die amphiphilen Pfropfpolymerisate ist das Eintopfverfahren, wobei in einem Reaktor zunächst die Pfropfgrundlage hergestellt, dann sofort die Pfropfkomponente auf einmal zugesetzt und dann polymerisiert oder während der Polymerisation nach Fortschritt der Reaktion zudosiert wird.

Die Pfropfpolymerisate besitzen K-Werte von mindestens 7 bis 300, bestimmt nach H. Fikentscher in 5 gew.-%iger wäßriger Lösung bei 25°C und pH 7. Die K-Werte der Pfropfpolymerisate liegen vorzugsweise in dem Bereich von 10 bis 200. Den angegebenen K-Werten entsprechen Molmassen M_{w} von 200 bis 10 Millionen. Die Molmassen M_{w} betragen vorzugsweise 500 bis 5 Millionen. Die Molmassen M_{w} wurden mit Hilfe der Lichtstreuung bestimmt.

Die so erhältlichen amphiphilen Pfropfpolymerisate werden als Verfestigungsmittel für Papier, als Fixiermittel für wasserlösliche und wasserunlösliche Störstoffe bei der Herstellung von Papier, als Dispergiermittel für anorganische und organische Pigmente, Farbstoffe, Beton und Pflanzenschutzmittel, als Waschmitteladditiv, als Fußbodenpflegemittel, als Schutzkolloid für wäßrige Polymerdisperisonen, als Verdicker für Kosmetikformulierungen, als Conditioner für Hautpflegemittel und als Bestandteil von haarkosmetischen Zubereitungen und von kosmetischen Zubereitungen für die Mundpflege verwendet.

Bei der Anwendung in Waschmitteln wirken die amphiphilen Pfropfpolymerisate als vergrauungsinhibierender Zusatz, fördern beim Waschen die Schmutzablösung (Soil-release Effekt) und inhibieren die Farbübertragung.

Die in den Beispielen angegebenen Teile sind Gewichtsteile. Die K-Werte wurden nach H. Fikentscher, Cellulose-Chemie, Band 13, 58 bis 64 und 71 gis 74 (1932) bei einer Temperatur von 25°C, einem pH-Wert von 7 und unter den in den Beispielen jeweils angegebenen Konzentrationen bestimmt. Die Molmassen M_{w} wurden nach der Methode der Lichtstreuung bestimmt.

### Beispiele

### Pfropfgrundlage A

In einem 2 1 fassenden Gefäß werden 823 g Wasser, 7,69 g 85 %ige Phosphorsäure und 6,72 g 50 %ige wäßrige Natronlauge vorgelegt. Der pH-Wert beträgt 6,5. Das Gefäß wird evakuiert, wobei zunächst ein Druck von 600 mbar und später von 500 mbar eingestellt wird. Der Inhalt des Gefäßes wird auf eine Temperatur von 80°C erhitzt. Innerhalb von 2 Stunden wird bei 80°C 245 g N-Vinylformamid (Zulauf 1) und in 3 Stunden 1,8 g 2,2'-Azobis(2-Methylpropionamidin)-dihydrochlorid gelöst in 117 g Wasser (Zulauf 2) zudosiert. Nach Zulaufende wird das Reaktionsgemisch noch 3 Stunden bei 80°C gerührt. Während der gesamten Reaktionszeit destilliert man insgesamt 149 g flüchtige Anteile ab, die kondensiert werden. Man erhält eine wäßrige Lösung mit einem Feststoffgehalt von 24,4 %. Der K-Wert des Poly-N-Vinylformamids beträgt 63,3 (gemessen in 1 %iger wäßriger Lösung). Die Molmasse M_{w} beträgt 70000.

### Pfropfgrundlage B

In einem 2 l fassenden Gefäß werden 100 g Wasser, 2,8 g 75 %ige Phosphorsäure und 1,9 g 50 %ige wäßrige Natronlauge vorgelegt. Der pH-Wert der Lösung beträgt 6,5. Die wäßrige Lösung wird unter einer Stickstoffatmosphäre auf 73°C erhitzt und der Druck auf 350 mbar eingestellt. Innerhalb von 2 Stunden dosiert man dann 200 g N-Vinylformamid (Zulauf 1) und innerhalb von 3 Stunden eine Lösung von 0,78 g 2,2'-Azobis(2-Methylpropionamidin)dihydrochlorid in 100 g Wasser (Zulauf 2) zu der auf 73°C erhitzten wäßrigen Lösung. Nach Beendigung der Initiatorzugabe wird das Reaktionsgemisch noch 3,5 Stunden bei 73°C auspolymerisiert. Während der gesamten Reaktionszeit destilliert man flüchtige Anteile aus dem Reaktionsgemisch und kondensiert sie. Die Menge des Kondensats beträgt insgesamt 400 g. Beim Abkühlen wird die Menge an Wasser, die abdestilliert wurde, durch Wasser ersetzt. Man erhält eine klare, farblose wäßrige Lösung mit einem Feststoffgehalt von 15,5 %. Der K-Wert des Poly-N-Vinylformamids beträgt 84,7 (bestimmt in 0,5 %iger wäßriger Lösung). Die Molmasse M_{w} des Polymeren beträgt 300000.

### Pfropfgrundlage C

In einem Reaktor legt man eine wäßrige Lösung von 1,8 g NaH₂PO₄ in 700 g Wasser vor und erhitzt die wäßrige Lösung im Stickstoffstrom auf eine Temperatur von 70°C. Sobald diese Temperatur erreicht ist, gibt man innerhalb von 2 Stunden eine wäßrige Lösung von 180 g N-Vinylformamid und 20 g N-vinylcaprolactam in 150 g Wasser sowie innerhalb von 3 Stunden eine wäßrige Lösung von 1,6 g 2,2'-Azobis(2-Methylpropionamidin)dihydrochlorid in 50 g Wasser gleichmäßig zu. Anschließend wird das Reaktionsgemisch eine Stunde bei 75°C gerührt. Man erhält eine wäßrige Copolymerisatlösung mit einem Feststoffgehalt von 17,3 %. Der K-Wert des Copolymerisats beträgt 78 (bestimmt bei einer Polymerkonzentration von 1 % in 5 %iger wäßriger Natriumchloridlösung). Die Molmasse M_{w} des Polymeren beträgt 200000.

### Beispiel 1

In einem Reaktor, der mit Rührer, Rückflußkühler und Dosiervorrichtungen versehen ist, werden 1003,6 g der 15,5 %igen wäßrigen Lösung von Pfropfgrundlage B vorgelegt und unter Stickstoff auf eine Temperatur von 85°C erhitzt. Sobald diese Temperatur erreicht ist, gibt man innerhalb von 3 Stunden 5,18 g Styrol und innerhalb von 4 Stunden eine wäßrige Lösung von 0,1 g 2,2'-Azobis(2-Methylpropionamidin)dihydrochlorid in 30 g Wasser gleichmäßig zu. Nach der Initiatorzugabe wird das Reaktionsgemisch noch 2 Stunden bei einer Temperatur von 85°C gerührt. Man erhält eine wäßrige Lösung eines Pfropfpolymerisats mit einem Feststoffgehalt von 17 %. Der K-Wert des Pfropfpolymeren beträgt 85,6 (gemessen in 1 %iger wäßriger Lösung), die Molmasse M_{w} 313000.

### Hydrolyse

### Beispiel 1.1

450 g der nach Beispiel 1 erhaltenen wäßrigen Polymerlösung werden unter Rühren auf eine Temperatur von 80°C erhitzt. Innerhalb von einer Stunde fügt man 86 g einer 50 %igen wäßrigen Natronlauge zu und rührt das Reaktionsgemisch noch 2 Stunden bei 80°C. Es wird dann abgekühlt und durch Zugaben von 85 g konzentrierter Salzsäure auf pH 7 eingestellt. Der Hydrolysegrad des mit Styrol gepfropften Poly-N-Vinylformamids beträgt 100 %. Die Molmasse des Polymeren beträgt 200000.

### Beispiel 1.2

400 g der nach Beispiel 1 erhaltenen wäßrigen Lösung des Pfropfpolymerisates werden unter Rühren auf eine Temperatur von 80° erhitzt. Sobald diese Temperatur erreicht ist, fügt man innerhalb von einer Stunde 57,5 g einer 50 %igen wäßrigen Natronlauge zu und rührt das Reaktionsgemisch noch 2 Stunden bei 80°C. Danach wird es abgekühlt und durch Zugabe von 57 g konzentrierter Salzsäure auf einen pH-Wert von 7 eingestellt. Der Hydrolysegrad des Pfropfpolymeren beträgt 75 %. Das Propfpolymer hat eine Molmasse M_{w} von 230000.

### Beispiel 2

In dem im Beispiel 1 beschriebenen Reaktor werden 1047 g der 15,5 %igen wäßrigen Lösung der Pfropfgrundlage B in einem Stickstoffstrom auf eine Temperatur von 85°C erhitzt. Sobald diese Temperatur erreicht ist, dosiert man innerhalb von 3 Stunden 9,0 g Styrol und innerhalb von 4 Stunden eine wäßrige Lösung von 0,18 g 2,2'-Azobis(2-methylpropionamidin)dihydrochlorid in 30 g Wasser gleichmäßig zu. Um das Reaktionsgemisch nachzupolymerisieren, gibt man anschließend 0,5 g 2,2'-Azobis(2-methylpropionamidin)dihydrochlorid zu und rührt das Reaktionsgemisch noch 2 Stunden bei 85°C. Man erhält eine wäßrige Lösung eines Pfropfpolymerisats mit einem Feststoffgehalt von 17,2 %. Der K-Wert des Pfropfpolymeren beträgt 85,6 (gemessen in 1 %iger wäßriger Lösung), die Molmasse M_{w} 325000.

### Hydrolyse

### Beispiel 2.1

500 g der nach Beispiel 2 erhaltenen wäßrigen Lösung des Pfropfpolymeren werden auf eine Temperatur von 80°C erhitzt und innerhalb von einer Stunden mit 97 g einer 50 %igen wäßrigen Natronlauge versetzt. Das Reaktionsgemisch wird anschließend noch 2 Stunden gerührt, abgekühlt und durch Zugabe von 99 g konzentrierter Salzsäure auf einen pH-Wert von 7 eingestellt. Der Hydrolysegrad des Pfropfpolymeren beträgt 100 %. Das hydrolysierte Pfropfpolymerisat hat eine Molmasse M_{w} von 212000.

### Beispiel 2.2

477,5 g der nach Beispiel 2 erhaltenen wäßrigen Lösung des Pfropfpolymeren werden auf eine Temperatur von 80°C erhitzt und innerhalb von einer Stunde mit 69,4 g einer 50 %igen wäßrigen Natronlauge versetzt. Das Reaktionsgemisch wird 2 Stunden bei 80°C gerührt, danach abgekühlt und durch Zugabe von 69 g konzentrierter Salzsäure auf einen pH-Wert von 7 eingestellt. Der Hydrolysegrad des Pfropfpolymeren beträgt 75 %. Das hydrolysierte Pfropfpolymerisat hat eine Molmasse M_{w} von 250000.

### Beispiel 3

In dem in Beispiel 1 angegebenen Reaktor erhitzt man 819,7 g der 24,4 %igen wäßrigen Lösung von Pfropfgrundlage A in einem Stickstoffstrom auf eine Temperatur von 85°C. Bei dieser Temperatur gibt man dann innerhalb von 3 Stunden 6,0 g Styrol und innerhalb von 4 Stunden eine wäßrige Lösung von 0,12 g 2,2'-Azobis-(2-Methylpropionamidin)dihydrochlorid in 30 g Wasser gleichmäßig zu. Zur Nachpolymerisation fügt man anschließend 0,5 g 2,2'-Azo-bis(2-Methylpropionamidin)dihydrochlorid zu und rührt das Reaktionsgemisch noch 2 Stunden bei einer Temperatur von 85°C. Man erhält eine wäßrige Lösung eines Pfropfpolymeren mit einem Feststoffgehalt von 23,4 % und einer Molmasse M_{w} von 74000.

### Beispiel 3.1

430 g der nach Beispiel 3 hergestellten wäßrigen Lösung des Pfropfpolymeren werden auf eine Temperatur von 80°C erhitzt und innerhalb von einer Stunde mit 118 g einer 50 %igen wäßrigen Natronlauge versetzt. Nach Zugabe der Natronlauge wird das Reaktionsgemisch noch 2 Stunden bei 80°C gerührt, danach abgekühlt und durch Zugabe von 108 g konzentrierter Salzsäure auf einen pH-Wert von 7 eingestellt. Der Hydrolysegrad des Pfropfpolymerisats beträgt 100 %. Das hydrolysierte Pfropfpolymerisat hat eine Molmasse M_{w} von 50000.

### Beispiel 3.2

375 g der nach Beispiel 3 erhaltenen wäßrigen Lösung einer Pfropfpolymerisats werden auf eine Temperatur von 80°C erhitzt und innerhalb von einer Stunde mit 77,3 g eine 50 %igen wäßrigen Natronlauge versetzt. Das Reaktionsgemisch wird anschließend noch 2 Stunden bei 80°C gerührt, danach abgekühlt und durch Zugabe von 75 g konzentrierter Salzsäure auf einen pH-Wert von 7 eingestellt. Der Hydrolysegrad des Pfropfpolymerisats beträgt 75 %. Das hydrolysierte Pfropfpolymerisat hat eine Molmasse M_{w} von 75000.

### Beispiel 4

In dem in Beispiel 1 beschriebenen Reaktor werden 901,6 g der 24,4 %igen wäßrigen Lösung von Pfropfgrundlage A vorgelegt und im Stickstoffstrom auf 85°C erhitzt. Bei dieser Temperatur werden dann innerhalb von 3 Stunden 11,0 g Styrol und innerhalb von 4 Stunden eine wäßrige Lösung von 0,22 g 2,2'-Azobis(2-Methylpropionamidin)dihydrochlorid in 30 g Wasser gleichmäßig zudosiert. Danach fügt man 0,5 g 2,2'-Azobis(2-Methylpropionamidin)dihydrochlorid zu und rührt das Reaktionsgemisch noch 2 Stunden zur Nachpolymerisation bei 85°C. Man erhält eine wäßrige Polymerlösung mit einem Feststoffgehalt von 23,5 %. Die Molmasse M_{w} des Pfropfpolymeren beträgt 77000.

### Beispiel 4.1

470 g der nach Beispiel 4 erhaltenen wäßrigen Lösung des Pfropfpolymerisats werden auf 80°C erhitzt und innerhalb einer Stunde mit 129 g einer 50 %igen wäßrigen Natronlauge versetzt. Das Reaktionsgemisch wird anschließend noch 2 Stunden gerührt, danach abgekühlt und durch Zugabe von 123 g konzentrierter Salzsäure auf einen pH-Wert von 7 eingestellt. Der Hydrolysegrad des Pfropfpolymeren beträgt 100 %. Die Molmasse M_{w} des hydrolysierten Pfropfpolymerisats beträgt 52000.

### Beispiel 4.2

425 g der nach Beispiel 4 erhaltenen wäßrigen Lösung des Pfropfpolymerisats werden auf 80°C erhitzt und innerhalb einer Stunde mit 87,6 g 50 %iger wäßriger Natronlauge versetzt. Das Reaktionsgemisch wird anschließend 2 Stunden gerührt, danach abgekühlt und durch Zugabe von 85 g konzentrierter Salzsäure auf einen pH-Wert von 7 eingestellt. Der Hydrolysegrad des Pfopfpolymerisats beträgt 75 %. Das hydrolysierte Pfropfpolymerisat hat eine Molmasse M_{w} von 49000.

### Anwendungstechnische Beispiele

Die Copolymeren werden bei der Herstellung von Papier als Fixiermittel für Störstoffe in Mengen von 0,30 bis 1,5 Gew.-%, bezogen auf Faserstoff, der Papiersuspension zugesetzt. Als Maß für die Fixierleistung wird die optische Durchlässigkeit des Filtrats bestimmt. Folgende Polymere wurden getestet:
Polymer 1: das nach Beispiel 1.2 hergestellte zu 75 % hydrolysierte und mit Styrol gepfropfte Polyvinylformamid.
Polymer 2: hergestellt nach Beispiel 4.1.
Polymer 3: Poly-diallyl-dimethylammoniumchlorid mit einer Molmasse M_{w} von 200000 (Vergleich gemäß Stand der Technik).
Polymer 4: handelsübliches, hochmolekulares, wasserlösliches Polymerisat auf Basis modifiziertem Polyethylenimin (Catiofast SF) Vergleich gemäß Stand der Technik.

### Beispiel 5

Eine wäßrige Faseraufschlämmung aus TMP (thermomechanische Pulpe) mit einer Stoffkonzentration von 2 % wurde in gleiche Anteile geteilt und jeweils mit einer wäßrigen Lösung von 5 % Huminsäure als Störstoff versetzt. Zu Proben dieser Pulpe gab man jeweils die in Tabelle 1 angegebenen Mengen an Polymer 1 sowie zusätzlich noch jeweils die gleiche Menge eines handelsüblichen hochmolekularen, kationischen Polyacrylamids als Flockungsmittel. Nach Durchmischen und Filtrieren des geflockten Papierstoffs wird die Extinktion des Filtrats bei 340 nm bestimmt.

**Tabelle 1:**

| eingesetztes Fixiermittel | | Menge Polymer, bezogen auf Papierstoff [%] | Extinktion des Filtrats bei 340 nm |
|---|---|---|---|
| Beispiele gemäß Erfindung | | | |
| a) | Polymer 1 | 0,30 | 0.793 |
| b) | Polymer 1 | 0,50 | 0.470 |
| c) | Polymer 1 | 0,80 | 0.256 |
| d) | Polymer 1 | 1,00 | 0.193 |
| e) | Polymer 1 | 1,25 | 0.183 |
| f) | Polymer 1 | 1,50 | 0.129 |

| Vergleichsbeispiele | | | |
|---|---|---|---|
| a) | Polymer 3 | 0,00 | 1.087 |
| b) | Polymer 3 | 0,30 | 0.549 |
| c) | Polymer 3 | 0.50 | 0.284 |
| d) | Polymer 3 | 0.80 | 0.222 |
| e) | Polymer 3 | 1.00 | 0.214 |
| f) | Polymer 3 | 1.25 | 0.189 |
| g) | Polymer 3 | 1.50 | 0.167 |

### Beispiel 6

Eine wäßrige Faseraufschlämmung aus TMP (thermomechanische Pulpe) mit einer Stoffkonzentration von 2 % wurde in gleiche Anteile geteilt und jeweils mit einer wäßrigen Lösung von A) 0,2 % Holzextrakt, B) 5 % Huminsäure und C) 15 % Ligninsulfonat als Störstoff versetzt. Zu Proben dieser Pulpe gab man jeweils die in Tabelle 2 angegebenen Mengen an zu prüfendem Polymer sowie zusätzlich noch jeweils die gleiche Menge eines handelsüblichen, hochmolekularen, kationischen Polyacrylamids als Flockungsmittel. Nach Durchmischen und Filtrieren des geflockten Papierstoffs wird die Extinktion des Filtrats bei 340 nm bestimmt.

**Tabelle 2:**

| eingesetztes Fixiermittel | | Menge Polymer, bezogen auf Papierstoff [%] | Extinktion des Filtrats bei 340 nm Störstoff | | |
|---|---|---|---|---|---|
| | | | A | B | C |
| Beispiele gemäß Erfindung | | | | | |
| a) | Polymer 1 | 0,30 | 0,434 | 0,656 | 0,831 |
| b) | Polymer 1 | 0,50 | 0,428 | 0,266 | 0,796 |
| c) | Polymer 1 | 0,80 | 0,311 | 0,163 | 0,857 |
| d) | Polymer 1 | 1,00 | 0,297 | 0,139 | 0,561 |
| e) | Polymer 1 | 1,25 | 0,236 | 0,123 | 0,411 |
| f) | Polymer 1 | 1,50 | 0,227 | 0,118 | 0,304 |

| Vergleichsbeispiele | | | | | |
|---|---|---|---|---|---|
| a) | Polymer 3 | 0,00 | 0,515 | 1,087 | 0,821 |
| b) | Polymer 3 | 0,30 | 0,411 | 0,549 | 0,882 |
| c) | Polymer 3 | 0,50 | 0,373 | 0,284 | 0,880 |
| d) | Polymer 3 | 0,80 | 0,332 | 0,222 | 0,871 |
| e) | Polymer 3 | 1,00 | 0,310 | 0,214 | 0,855 |
| f) | Polymer 3 | 1,25 | 0,283 | 0,189 | 0,847 |
| g) | Polymer 3 | 1,50 | 0,266 | 0,167 | 0,411 |

### Beispiel 7

Eine wäßrige Faseraufschlämmung aus TMP (thermomechanische Pulpe) mit einer Stoffkonzentraiton von 2 % wurde in gleiche Anteile geteilt und jeweils mit einer wäßrigen Lösung von A) 0,2 % Holzextrakt, B) 5 % Huminsäure und C) 15 % Ligninsulfonat als Störstoff versetzt. Zu Proben dieser Pulpe gab man jeweils die in Tabelle 3 angegebenen Mengen an zu prüfendem Polymer sowie zusätzlich noch jeweils die gleiche Menge eines handelsüblichen hochmolekularen, kationischen Polyacrylamids als Flockungsmittel. Nach Durchmischen und Filtrieren des geflockten papierstoffs wird die Extinktion des Filtrats bei 340 nm bestimmt.

**Tabelle 3:**

| eingesetztes Polymer | | Menge Polymer, bezogen auf Papierstoff [%] | Extinktion des Filtrats bei 340 nm Störstoff | | |
|---|---|---|---|---|---|
| | | | A | B | C |
| Beispiele gemäß Erfindung | | | | | |
| a) | Polymer 2 | 0,30 | 0,442 | 0,642 | 0,862 |
| b) | Polymer 2 | 0,50 | 0,425 | 0,396 | 0,854 |
| c) | Polymer 2 | 0,80 | 0,375 | 0,191 | 0,853 |
| d) | Polymer 2 | 1,00 | 0,318 | 0,132 | 0,850 |
| e) | Polymer 2 | 1,25 | 0,291 | 0,112 | 0,788 |
| f) | Polymer 2 | 1,50 | 0,264 | 0,109 | 0,352 |

| Vergleichsbeispiele | | | | | |
|---|---|---|---|---|---|
| a) | Polymer 4 | 0,00 | 0,561 | 1,162 | 0,862 |
| b) | Polymer 4 | 0,30 | 0,484 | 0,511 | 0,972 |
| c) | Polymer 4 | 0,50 | 0,426 | 0,224 | 0,853 |
| d) | Polymer 4 | 0,80 | 0,393 | 0,175 | 0,797 |
| e) | Polymer 4 | 1,00 | 0,382 | 0,131 | 0,718 |
| f) | Polymer 4 | 1,25 | 0,301 | 0,110 | 0,311 |
| g) | Polymer 4 | 1,50 | 0,200 | 0,111 | 0,289 |

## Patentansprüche

1. Amphiphile Pfropfpolymerisate auf Basis von N-Vinylcarbonsäureamid-Einheiten enthaltenden Pfropfgrundlagen, **dadurch gekennzeichnet, daß** sie erhältlich sind durch radikalische Polymerisation von Monomermischungen aus
(a) Styrol, C₁- bis C₂-Alkylstyrol und/oder Vinyltoluol und gegebenenfalls
(b) anderen damit copolymerisierbaren monoethylenisch ungesättigten Monomeren
auf ein Polymerisat, das mindestens 5 Gew.-% Einheiten der Formel 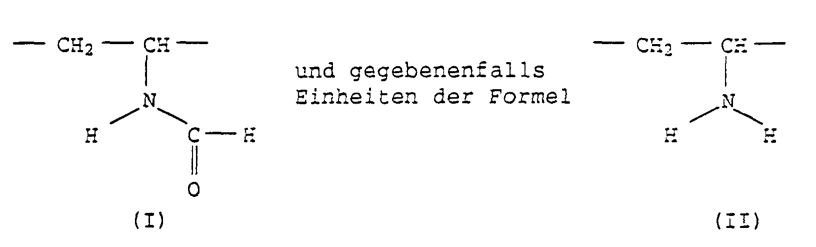 enthält, als Pfropfgrundlage, im Gewichtsverhältnis 1:99 bis 99:1 und anschließende 5 bis 100%ige Abspaltung der Formylgruppen unter Bildung von Aminogruppen aus den Pfropfpolymerisaten.

2. Amphiphile Pfropfpolymerisate nach Anspruch 1, **dadurch gekennzeichnet, daß** auf eine Pfropfgrundlage aus einem Homopolymerisat aus N-Vinylformamid und aus einem Copolymerisat aus N-Vinylformamid und N-Vinylcaprolactam Monomermischungen aus
(a) 5 bis 100 Gew.-% Styrol, C₁- bis C₂-Alkylstyrol und/oder Vinyltoluol und
(b) 0 bis 95 Gew.-% anderen copolymerisierbaren monoethylenisch ungesättigten Monomeren
aufgepfropft sind.

3. Amphiphile Pfropfpolymerisate nach Anspruch 1 oder 2, **dadurch gekennzeichnet, daß** auf eine Pfropfgrundlage aus Poly-N-Vinylformamid Styrol aufgepfropft ist.

4. Verfahren zur Herstellung der amphiphilen Pfropfpolymerisate nach den Ansprüchen 1 bis 3, **dadurch gekennzeichnet, daß** man Monomermischungen aus
(a) Styrol, C₁- bis C₂-Alkylstyrol und/oder Vinyltoluol und gegebenenfalls
(b) anderen damit copolymerisierbaren monoethylenisch ungesättigten Monomeren
radikalisch in Gegenwart von Polymerisaten, die mindestens 5 Gew.-% N-Vinylformamid-Einheiten enthalten, als Pfropfgrundlage im Gewichtsverhältnis 1 : 99 bis 99 : 1 polymerisiert und im Anschluß an die Pfropfpolymerisation 5 bis 100% der Formylgruppen aus den Vinylformamid-Einheiten der Formel I unter Bildung von Aminogruppen abspaltet.

5. Verfahren nach Anspruch 4, **dadurch gekennzeichnet, daß** man die Pfropfpolymerisation in wäßriger Lösung durchführt.

6. Verwendung der amphiphilen Pfropfpolymerisate nach den Ansprüchen 1 bis 3 als Verfestigungsmittel für Papier, als Fixiermittel für wasserlösliche und wasserunlösliche Störstoffe bei der Herstellung von Papier, als Dispergiermittel für anorganische und organische Pigmente, Farbstoffe, Beton und Pflanzenschutzmittel, als Waschmitteladditiv, als Beschichtungsmaterial für Düngemittel und Pflanzenschutzmittel, als Fußbodenpflegemittel, als Schutzkolloid für wäßrige Polymerdispersionen, als Verdicker für Kosmetikformulierungen, als Conditioner für Hautpflegemittel und als Bestandteil von haarkosmetischen Zubereitungen und von kosmetischen Zubereitungen für die Mundpflege.

## Claims

1. An amphiphilic graft polymer based on a graft base containing N-vinylcarboxamide units, which is obtainable by free radical polymerization of a monomer mixture comprising
(a) styrene, C₁- or C₂-alkylstyrene and/or vinyltoluene and, if required,
(b) other monoethylenically unsaturated monomers copolymerizable therewith
onto a polymer which contains at least 5% by weight of units of the formula 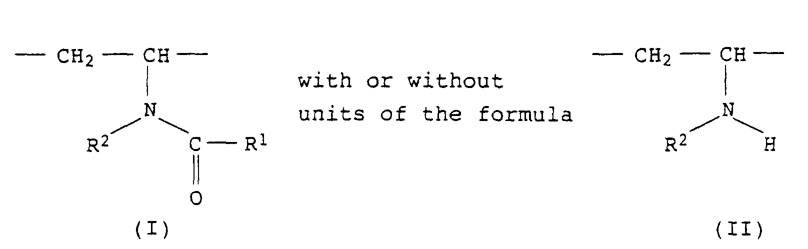 as the graft base, in a weight ratio of from 1 : 99 to 99 : 1, and subsequent elimination of from 5 to 100% of the formyl groups, with formation of amino groups, from the graft polymer.

2. An amphiphilic graft polymer as claimed in claim 1, wherein there are grafted, onto a graft base comprising a homopolymer of N-vinylformamide and a copolymer of N-vinylformamide and N-vinylcaprolactam, monomer mixtures containing
(a) from 5 to 100% by weight of styrene, a C₁- to C₂-alkylstyrene and/or vinyltoluene and
(b) from 0 to 95% by weight of other copolymerizable monoethylenically unsaturated monomers.

3. An amphiphilic graft polymer as claimed in claim 1 or 2, wherein styrene is grafted onto a graft base of poly-N-vinylformamide.

4. A process for preparing an amphiphilic graft polymer as claimed in any of claims 1 to 3, which comprises subjecting monomer mixtures of
(a) styrene, a C₁- to C₂-alkylstyrene and/or vinyltoluene with or without
(b) other monoethylenically unsaturated monomers copolymerizable therewith
to free-radical polymerization in the presence of polymers containing at least 5% by weight of N-vinylformamide units as graft base, in a weight ratio of from 1 : 99 to 99 : 1, and, after the graft polymerization, cleaving off 5 to 100% of the formyl groups from the vinylformamide units of the formula I with the formation of amino groups.

5. A process as claimed in claim 4, wherein the graft polymerization is conducted in aqueous solution.

6. The use of an amphiphilic graft polymer as claimed in any of claims 1 to 3 as a strength enhancer for paper, as a fixing agent for water-soluble and water-insoluble contaminants in papermaking, as a dispersant for organic and inorganic pigments, dyes, concrete and crop protection agents, as a detergent additive, as a coating material for fertilizers and crop protection agents, as a floor care agent, as a protective colloid for aqueous polymer dispersions, as a thickener for cosmetics formulations, as a conditioner for skincare compositions and as a constituent of cosmetic hair preparations and of cosmetic preparations for oral care.

## Revendications

1. Polymères greffés amphiphiles élaborés à partir de bases contenant des unités vinylcarboxamide, **caractérisés en ce qu'**ils peuvent être obtenus par polymérisation radicalaire de mélanges de monomères constitués de
(a) styrène, alkylstyrène en C₁ à C₂ et/ou vinyltoluène et,
le cas échéant,
(b) d'autres monomères monoéthyléniquement insaturés copolymérisables avec ces derniers
sur un polymère qui contient au moins 5% en poids d'unités de formule: 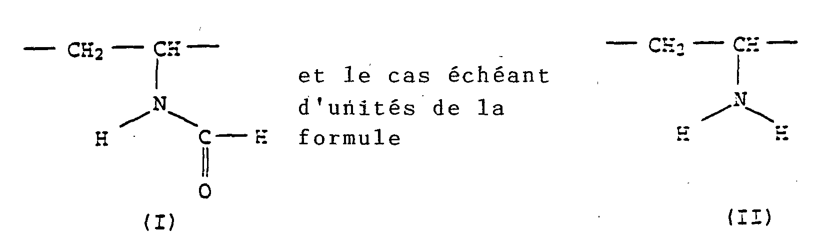 comme base de greffage, dans un rapport pondéral de 1/99 à 99/1 et séparation suivante de 5 à 100% des groupes formyle avec formation de groupes amino, des polymères greffés.

2. Polymères greffés amphiphiles selon la revendication 1, **caractérisés en ce que** sont greffés, sur une base de greffage constituée d'un homopolymère de N-vinylformamide, d'un copolymère de N-vinylformamide et de N-vinylcaprolactame, des mélanges de monomères constitués de
(a) 5 à 100 % en poids de styrène, d'alkylstyrène en C₁ à C₂ et/ou de vinyltoluène et
(b) 0 à 95% en poids d'autres monomères monoéthyliquement insaturés, copolymérisables.

3. Polymères greffés amphiphiles selon la revendication 1 ou 2, **caractérisés en ce que** du styrène est greffé sur une base de greffage constituée de poly-N-vinylformamide.

4. Procédé de fabrication des polymères greffés amphiphiles selon les revendications 1 à 3, **caractérisé en ce que** l'on polymérise des mélanges de monomères constitués de
(a) styrène, alkylstyrène en C₁ à C₂ et/ou vinyltoluène et, le cas échéant,
(b) d'autres monomères mono-éthyléniquement insaturés copolymérisables avec ces derniers
de manière radicalaire en présence de polymères qui contiennent au moins 5% en poids d'unités de N-vinylformamide comme base de greffage dans un rapport pondéral de 1/99 à 99/1 et que l'on sépare à la suite de la polymérisation greffée 5 à 100% des groupes formyle des unités de vinylformamide de la formule I avec formation de groupes amino.

5. Procédé selon la revendication 4, **caractérisé en ce que** l'on effectue la polymérisation greffée en solution aqueuse.

6. Utilisation des polymères greffés amphiphiles selon les revendications 1 à 3 comme produits de renforcement pour le papier, comme fixateurs pour les matières hydrosolubles et non hydrosolubles gênantes dans la fabrication du papier, comme produits dispersants pour les pigments inorganiques et organiques, colorants, produits de protection du béton et produits phytosanitaires, comme additifs aux produits de lavage, comme matières de revêtement pour des fertilisants et des produits phytosanitaires, comme produits d'entretien des sols, comme colloïdes protecteurs pour des dispersions de polymères aqueuses, comme épaississants pour les formulations cosmétiques, comme agents de conditionnement pour les produits d'entretien de la peau et comme constituants de préparations cosmétiques capillaires et de préparations cosmétiques pour les soins buccaux.
